# EUROPÄISCHE PATENTSCHRIFT

(11) **EP 1 771 404 B1**
(45) Veröffentlichungstag und Bekanntmachung des Hinweises auf die Patenterteilung: **03.03.2010**
(21) Anmeldenummer: 05777860.7
(22) Anmeldetag: 18.07.2005
(51) Int. Cl.: C07C 45/67, C07C 49/587

(54) **VERBESSERTES VERFAHREN ZUR HERSTELLUNG VON (E,Z)-7,8-CYCLOHEXADECEN-1-ON**
IMPROVED METHOD FOR THE PRODUCTION OF (E,Z)-7,8-CYCLOHEXADECENE-1-ON
PROCEDE AMELIORE DE PRODUCTION DE (E,Z)-7,8-CYCLOHEXADECEN-1-ONE

(30) Priorität: 21.07.2004 DE 102004035389
(43) Veröffentlichungstag der Anmeldung: 11.04.2007
(73) Patentinhaber: Symrise GmbH & Co. KG, 37603 Holzminden (DE)
(72) Erfinder: SURBURG, Horst, 37603 Holzminden (DE); DILK, Erich, 37603 Holzminden (DE); RECKZIEGEL, Aurelia, 41540 Dormagen (DE); KUHN, Walter, 37603 Holzminden (DE)
(74) Vertreter: Eisenführ, Speiser & Partner
(86) Internationale Anmeldenummer: PCT/EP2005/053454
(87) Internationale Veröffentlichungsnummer: WO 2006/008286

(56) Entgegenhaltungen:
- WO-A-2005/063670
- US-A1- 2002 055 453
- MATHUR H H ET AL: "MACROCYCLIC MUSK COMPOUNDS-IX. NEW SYNTHESES OF CYCLOHEXADECENONE AND CYCLOHEXADECANONE FROM ALEURITIC ACID" TETRAHEDRON, ELSEVIER SCIENCE PUBLISHERS, AMSTERDAM, NL, Bd. 21, 1965, Seiten 1537-1540, XP002230767 ISSN: 0040-4020 in der Anmeldung erwähnt
- P.A. GRIECO ET AL.: "Remote double bond migration via rhodium catalysis: a novel enone transposition" J.AM.CHEM.SOC., Bd. 98, Nr. 22, 1976, Seiten 7102-7104, XP002354942 in der Anmeldung erwähnt

## Beschreibung

Die vorliegende Erfindung betrifft ein verbessertes Verfahren zur Herstellung von (E,Z)-7,8-Cyclohexadecen-1-on.

DE 103 61 524 offenbart eine Mischung von (E,Z)-7-Cyclohexadecen-1-on und (E,Z)-8-Cyclohexadecen-1-on, für die ein kräftiger, sauberer und komplexer Moschusgeruch angegeben wird. Die Mischung weist einen eleganten, exaltierenden und kristallinen Moschusgeruch auf und mit ihr können zum Beispiel an Moschus Ambrette erinnernde Geruchseffekte erzielt werden. Das (E,Z)-7,8-Cyclohexadecen-1-on-Isomerengemisch stellt somit eine interessante und wertvolle Riech- und Aromastoffmischung dar.

Für die Herstellung von (E,Z)-7,8-Cyclohexadecen-1-on wird in DE 103 61 524 ausgehend von einem Gemisch von 1,8- und 1,9-Cyclohexadecandion ein 2-stufiger Prozess angegeben, wobei zunächst eine partielle Reduktion und anschließend eine saure Dehydratisierung durchgeführt werden. Es resultiert gemäß Beispiel 1 der DE 103 61 524 ein Reaktionsgemisch bestehend aus 53% (E,Z)-7,8-Cyclohexadecen-1-on, 22% nicht umgesetztem 1,8/1,9-Cyclohexadecandion und 22% Cyclohexadecadien. Dieses Gemisch wird durch fraktionierte Destillation getrennt. Während das 1,8/1,9-Cyclohexadecandion in den Prozess zurückgeführt werden kann, muss das Cyclohexadecadien verworfen werden. Das (E,Z)-7,8-Cyclohexadecen-1-on wird nach diesem Herstellverfahren in einer destillierten Ausbeute von lediglich 35% erhalten.

DE 103 61 524 gibt außerdem ein Verfahren zur Herstellung von (E,Z)-7-Cyclohexadecen-1-on durch Olefinmethathese an, wobei das hierfür benötigte 1,17-Octadecadien-8-on in einem aufwendigen mehrstufigen Prozess hergestellt werden muss.

Die Synthese eines Gemisches von (E)-7-Cyclohexadecen-1-on und (E)-8-Cyclohexadecen-1-on ist in Tetrahedron, 1965, 21, 1537 beschrieben. Aleuritinsäure (9,10,16-Trihydroxypalmitinsäure) wird als Isopropylidenderivat geschützt, zur Dicarbonsäure oxidiert, hydrobromiert und verestert. Aus dem resultierenden Diester erhält man nach Brom-Eliminierung über weitere Stufen ein Gemisch von (E)-7-Cyclohexadecen-1-on und (E)-8-Cyclohexadecen-1-on.

Gesucht wird daher ein einfaches und effektives Verfahren zur Herstellung von (E,Z)-7,8-Cylcohexadecen-1-on, das insbesondere auch für eine großtechnische Herstellung geeignet ist.

Überraschenderweise hat sich gezeigt, dass ein (E,Z)-7,8-Cyclohexadecen-1-on-Isomerengemisch in einfacher Weise mit hoher Selektivität durch Isomerisierung von (E,Z)-8-Cyclohexadecen-1-on erhalten werden kann. Die entsprechende Umsetzung ist nachfolgend schematisch wiedergegeben:

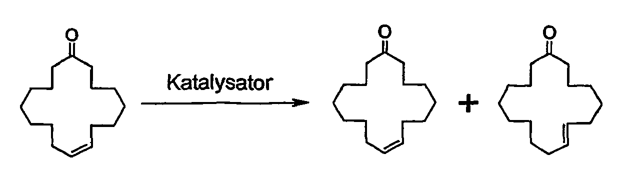

Während Isomerisierungen von aliphatischen Olefinen, wie zum Beispiel Allylumlagerungen, umfangreich in der Literatur beschrieben sind, liegen Literaturhinweise zur Verschiebung von Doppelbindungen in makrocyclischen Ringsystemen um lediglich ein Kohlenstoffatom nicht vor.

Für makrocyclische Alkadiene mit einer Ringgröße von 12 bis 22 Ringatomen wurde von A.J. Hubert und J. Dale in Journal of the Chemical Society, 1963, 4091-4096 eine Isomerisierung der Doppelbindung mit Triethylboran bei 200°C beschrieben. Unter diesen Bedingungen resultiert jedoch ein Doppelbindungsisomerengemisch mit einer statistischen Verteilung der theoretisch möglichen Doppelbindungsisomeren. Von den selben Autoren wurde im Journal of the Chemical Society, 1965, 3118-3126 ebenfalls über eine statistische Produktverteilung bei der Isomerisierung von makrocyclischen Alkadienen mit Kalium-tert.-butylat berichtet.

P. A. Grieco et al. beschreiben im Journal of the American Chemical Society, 1976,98, 7102-7104 für α-alkylsubstituierte Cycloalkenone mit 6 bis 8 Ringatomen eine Wanderung der Doppelbindung über den Ring unter Ausbildung des stabileren α,β-ungesättigten Isomeren durch 3-stündiges Erhitzen mit Rhodium-III-chlorid Trihydrat auf 100°C beschrieben.

Es ist daher völlig überraschend und nicht zu erwarten gewesen, dass ausgehend von (E,Z)-8-Cyclohexadecen-1-on eine selektive Verschiebung der Doppelbindung um lediglich ein Kohlenstoffatom erreicht werden kann. Im erfindungsgemäßen Verfahren wird das (E,Z)-8-Cyclohexadecen-1-on so mit
(i) einer Säure ausgewählt aus der Gruppe bestehend aus Schwefelsäure und Sulfonsäuren und sauren Kationenaustauschern, oder
(ii) einem Katalysator enthaltend ein Element der VIII. Nebengruppe kontaktiert,
dass es teilweise unter Bildung des (E,Z)-7,8-Cyclohexadecen-1-on-Isomerengemisches isomerisiert.

Das resultierende (E,Z)-7,8-Cyclohexadeoen-1-on-Isomerengemisch enthält dann ca. 35-40% (E,Z)-7- und ca. 60% (E,Z)-8-Isomeres. Die Bildung anderer Isomerer erfolgt entweder gar nicht oder nur in geringem Maße. Eine Isomerisierung mit einer statistischen Verteilung der Doppelbindungsisomeren wird nicht beobachtet.

### (i) Säuren zur Verwendung im erfindungsgemäßen Verfahren:

Eingesetzt werden Schwefelsäure oder Sulfonsäuren als anorganische Protonensäure. Beispiele für Sulfonsäuren sind p-Toluolsulfonsäure, Benzolsulfonsäure, Methansulfonsäure, Trifluormethansulfonsäure. Bevorzugte saure Katalysatoren sind Trifluormethansulfonsäure und p-Toluolsulfonsäure. Die zu verwendende Menge hängt von der jeweiligen Sulfonsäure ab. So ist beim Einsatz von Trifluormethansulfonsäure bereits 1 Gewichtsprozent (bezogen auf das eingesetzte (E,Z)-8-Cyclohexadecen-1-on) ausreichend, während von der p-Toluolsulfonsäure vorzugsweise 10 bis 30 Gewichtsprozent (bezogen auf das eingesetzte (E,Z)-8-Cyclohexadecen-1-on) eingesetzt werden.

Alternativ werden saure Kationenaustauschern als saure Festbettkatalysator eingesetzt. Zur Gruppe der sauren Kationenaustauscher gehören insbesondere Kationenaustauscher auf der Basis von Polymerisationskunstharzen mit unterschiedlicher Vernetzung, makroporöser Struktur und aktiven Gruppen verschiedener Säurestärke als schwach bis stark saure Ionenaustauscher auf Kunstharzbasis sind insbesondere Lewatite^{®} (Bayer) und Amberlite^{®} (Rohm und Haas) zu nehmen. Des Weiteren eingesetzt werden können Montmorillonite, wie z. B. die K-Katalysatoren (Bezeichnung der Firma Südchemie für speziell säurebehandelte Montmorillonite).

Die durch eine Säure katalysierte Isomerisierung kann sowohl ohne Lösungsmittel als auch unter Verwendung eines inerten Lösungsmittels wie zum Beispiel Cyclohexan, Toluol oder Xylol durchgeführt werden, wobei letztere Variante besonders bevorzugt ist.

Um die säurekatalysierte Isomerisierung mit zufriedenstellender Geschwindigkeit durchzuführen, wird vorzugsweise eine Reaktionstemperatur oberhalb 80°C, bevorzugt oberhalb 100°C und inbesondere bevorzugt oberhalb 120°C gewählt. Die Reaktionszeit ist von der Reaktionstemperatur und den sonstigen Reaktionsbedingungen abhängig. So werden z. B. bei Umsetzung mit 20 Gewichtsprozent (bezogen auf das eingesetzte (E,Z)-8-Cyclohexadecen-1-on) p-Toluolsulfonsäure bei 115°C und Verwendung von Toluol als Lösungsmittel 30-40 Stunden für die Einstellung des Isomerengleichgewichts benötigt.

Wird die ansonsten gleiche Reaktion bei 140°C in Xylol als Lösungsmittel durchgeführt, so sind bereits 3-4 Stunden ausreichend.

### (ii) Metall-Katalysatoren (Katalysatoren enthaltend ein Element der VIII. Nebengruppe) zur Verwendung im erfindungsgemäßen Verfahren:

Als Katalysatoren für die Isomerisierung können gemäß obiger Alternative (ii) auch Katalysatoren enthaltend ein Element der VIII. Nebengruppe verwendet werden. Als Elemente der VIII. Hauptgruppe können dabei insbesondere Ruthenium, Rhodium, Palladium, Osmium, Iridium und Platin eingesetzt werden, wobei Ruthenium, Rhodium, Palladium und Iridium besonders bevorzugt sind. Die genannten Katalysatoren können in elementarer, metallischer Form verwendet werden, wobei sie im allgemeinen auf einem Träger aufgebracht sind. Bevorzugt hierfür sind Träger-Materialien wie Aktivkohle, Aluminiumoxid oder Siliciumoxid. Die Konzentration der Katalysatoren auf dem Trägermaterial liegt dabei vorzugsweise zwischen 5 und 10%.

Zur Aktivitäts- und/oder Selektivitätssteigerung sind die Elemente der VIII. Nebengruppe bevorzugt mit Liganden komplexiert. In den Übergangsmetallverbindungen liegen die Elemente der VIII. Nebengruppe im allgemeinen formal als nullwertig beziehungsweise einfach, zweifach oder dreifach positiv geladen vor. Als Gegenionen können beispielsweise Chlorid, Bromid, Jodid, Sulfat, Nitrat, Sulfonat, oder Borat verwendet werden. Als Liganden sind zum Beispiel Acetonitril, Benzonitril, Diethylether, Kohlenmonoxid, Tetrahydrofuran, Wasserstoff, Amine, Ketone, Phosphane, Essigsäureethylester, Dimethylsulfoxid, Dimethylformamid oder Hexamethyl-phosphorsäuretriamid geeignet.

Zusammengefasst sind also erfindungsgemäße Verfahren bevorzugt, wobei das Element der VIII. Nebengruppe vorliegt
(a) in elementarer Form,
(b) in komplexierter oder unkomplexierter Form als Salz, wobei es eine formal ein- bis dreiwertige Oxidationsstufe besitzt,
   oder
(c) als Komplexverbindung, wobei es formal nullwertig ist

Beispielhaft seien folgende Katalysatoren genannt.

### Rhodiumkatalysatoren:

Rhodium(III)-bromid Hydrat, Rhodium(III)-chlorid, Rhodium(III)-chlorid Hydrat, Rhodium(III) jodid Hydrat, Rhodium(III)-nitrat, Rhodium(III)-phosphat, Rhodium(III)-sulfat, Rhodium(II)-acetat dimer, Rhodium(III)acetonylacetonat, Rhodium(I)-bis-(1,5-cyclooctadien)-tetrafluoroborat Hydrat, Rhodium(I)-bis-(1,5-cyclooctadien)-acetylacetonat, Rhodium(I)-bis-(1,5-cyclooctadien)-chlorid dimer, Rhodium(I)-bis-(1,5-cyclooctadien)-trifluormethansulfonat dimer, Rhodium(I)-[1,4-bis-(diphenylphosphino)-butan]-(1 c,5c-cyclooctadien)-tetrafluoroborat, Rhodium(I)-[1,4-bis-(diphenylphosphino)-butan]-((2,5-norbornandien)-tetrafluoroborat, Rhodium(I)-(2,5-norbomandien)-perchlorat, Rhodium(I)-bis-(triphenylphosphin)-carbonyl-chlorid, Rhodium(II)-trifluoracetat dimer, Rhodium(I)-tris-(triphenylphosphin)-bromid, Rhodium(I)-tris-(triphenylphosphin)-chlorid, Rhodium(I)-dicarbonyl-acetylacetonat, Rhodium(I)-dicarbonyl-chlorid dimer,

### Rutheniumkatalysatoren:

Ruthenium(III)-bromid Hydrat, Ruthenium(III)-chlorid, Ruthenium(III)-chlorid Hydrat, Ruthenium(III)-jodid, Ruthenium carbonyl, Ruthenium(I)-acetat polymer, Ruthenium(III)-acetonylacetonat, Ruthenium(II)-(1,5-cyclooctadien)-chlorid polymer, Ruthenium(II)-tris-(triphenylphosphin)-chlorid, Ruthenium(II)-tricarbonyl-chlorid dimer, Ruthenium(II)-carbonyldihydrido-tris-(triphenylphosphin), Ruthenium(III)-2,4-pentanedionat,

### Palladiumkatalysatoren:

Palladium (II)-acetat, Palladium(II)-acetonylacetonat, Palladium(II)-bis-(acetonitril)-chlorid, Palladium(II)-bis-(benzonitril)-chlorid, Palladium(II)-[1,2-bis-(diphenylphosphino)-ethan]-chlorid, Palladium(II)-bis (tricyclohexylphosphin)-chlorid, Palladium(II)-bis-(triphenylphosphin)-chlorid, Palladium(II)-bis-(triphenylphosphin)-bromid, Palladium(II)-bromid, Palladium(II)-chlorid, Palladium(II)-diammin-chlorid, Palladium(II)-jodid, Palladium(II)-nitrat, Palladium(II)-2,5-norbornadien-chlorid, Palladium(II)-sulfat, Palladium(II)-tetrammin-chlorid, Palladium(II)-[1,1'-ferrocenylbis(diphenylphosphan)]-dichlorid Dichlormethan, Palladium auf Aktivkohle, Palladium auf Aluminiumoxid,

### Iridiumkatalysatoren:

Iridiumacetat, Iridium(III)-acetylacetonat, Iridium(I)-bis-(triphenylphosphin)-carbonyl-chlorid, Iridium(III)-bromid Hydrat, Iridiumcarbonyl, Iridium(III)-chlorid, Iridium(III)-chlorid Hydrat, Iridium(I)-(1,5-cyclooctadien)-acetylacetonat.

Besonders bevorzugte Katalysatoren sind beispielsweise Rhodium(I)-bis-(triphenylphosphin)-carbonyl-chlorid, Palladium(II)-bis-(benzonitril)-chlorid, Ruthenium(II)-tris-(triphenylphosphin)-chlorid, Iridium(I)-bis-(triphenylphosphin)-carbonylchlorid und Palladium auf Aktivkohle.

Die durch einen solchen Metall-Katalysator katalysierte Isomerisierung wird vorzugsweise im Temperaturbereich von 40 bis 250°C durchgeführt, wobei bei niedrigen Temperaturen längere Reaktionszeiten notwendig sind und bei höheren Temperaturen in gewissem Maße Zersetzungsreaktionen auftreten können. Ein besonders bevorzugter Temperaturbereich liegt zwischen 80 und 180°C.

Zur Isomerisierung werden Katalysator-Konzentrationen ≥ 0,01 Gewichtsprozent (bezogen auf das eingesetzte (E,Z)-8-Cyclohexadecen-1-on) eingesetzt, wobei bevorzugte Konzentrationen im Bereich von 0,02 - 3 Gew.-% und besonders bevorzugte Konzentrationen im Bereich von 0,05 - 0,15 Gew.-% liegen; eine Konzentration von 0,1 Gewichtsprozent ist ganz besonders bevorzugt.

Im Falle des elementaren, gegebenenfalls auf einen Träger aufgebrachten, Palladiums liegt die Konzentrationen an Palladium bevorzugt im Bereich von 0,01 bis 0,15 Gew.-% und besonders bevorzugt im Bereich von 0,02 bis 0,08 Gew.-%, bezogen auf die Masse des an eingesetztem (E,Z)-8-Cyclohexadecen-1-on.

Die durch einen der genannten Katalysatoren katalysierte Isomerisierung kann sowohl unter Verwendung eines inerten Lösungsmittels wie zum Beispiel Toluol, Xylol, Cyclohexan als auch ohne Lösungsmittel durchgeführt werden, wobei letztere Variante besonders bevorzugt ist.

### Beispiele

Das in den Isomerisierungsbeispielen eingesetzte Eduktmaterial enthält 98% (E,Z)-8-Cyclohexadecen-1-on, wobei 67% E-Isomer und 31% Z-Isomer enthalten sind.

### Beispiele 1-6: Umisomerisierung mit Säurekatalysatoren.

### Beispiel 1

### Reaktionsbedingungen: Katalysator: p-Toluolsulfonsäure (20 Gew.-%), Lösungsmittel: Xylol; Temperatur: 140°C, Reaktionszeit: 4 Stunden

360 g Eduktmaterial, 72 g p-Toluolsulfonsäure-Monohydrat und 1400 mL Xylol werden 4 Stunden bei 140°C erhitzt, wobei zunächst 7,4 g Wasser, aus dem Monohydrat stammend, am Wasserabscheider abgetrennt werden. Nach dem Reaktionsende wird bei 60°C mit 1000 g 5%-iger Natriumhydrogencarbonatlösung gewaschen. Zur Verbesserung der Phasentrennung werden 300 g Kochsalzlösung zugegeben. Man trennt 1320 g Wasserphase ab. Die organische Phase wird am Rotationsverdampfer eingeengt, wonach 418 g Rückstand verbleiben, die an einer 30cm-Füllkörperkolonne destilliert werden. Bei einem Druck von 0,82 mbar wird bei einer Siedetemperatur von 130°C eine Hauptfraktion von 288 g Produkt erhalten, die einer Ausbeute von 80% der Theorie entsprechen. Das Produkt weist folgende Zusammensetzung auf (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 38,4 | 26,9 |
| Z-Isomer | 24,6 | 9,2 |
| Summe | 63 | 36,1 |

### Beispiel 2

### Reaktionsbedingungen: Katalysator: Methansulfonsäure (20 Gew.-%), Lösungsmittel: Xylol; Temperatur: 140°C, Reaktionszeit: 4 Stunden

50 g (E,Z)-8-Cyclohexadecen-1-on, 10 g Methansulfonsäure und 200 mL Xylol werden 4 Stunden bei 140°C erhitzt. Die Aufarbeitung erfolgt analog den Angaben in Beispiel 1. Nach Destillation werden 30 g (Ausb.: 60 % d.Th.) Produkt mit folgender Zusammensetzung erhalten (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 43,5 | 27,8 |
| Z-Isomer | 12,0 | 8,0 |
| Summe | 55,5 | 35,8 |

### Beispiel 3

### Reaktionsbedingungen: Katalysator: Trifluormethansulfonsäure (1 Gew.-%), Lösungsmittel: Xylol; Temperatur: 120°C, Reaktionszeit: 13 Stunden

30 g (E,Z)-8-Cyclohexadecen-1-on, 0,3 g Trifluormethansulfonsäure und 120 mL Xylol werden 13 Stunden bei 120°C erhitzt. Die Aufarbeitung erfolgt analog den Angaben in Beispiel 1. Nach Destillation werden 24,3 g (Ausb.: 81 % d.Th.) Produkt mit folgender Zusammensetzung erhalten (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 44,6 | 30,8 |
| Z-Isomer | 14,2 | 8,4 |
| Summe | 58,8 | 39,2 |

### Beispiel 4:

### Reaktionsbedingungen: Katalysator: Schwefelsäure (3 Gew.-%); kein Lösungsmittel; Temperatur: 120°C, Reaktionszeit: 8 Stunden

50 g (E,Z)-8-Cyclohexadecen-1-on und 1,5 g konzentrierte Schwefelsäure werden 8 Stunden bei 120°C erhitzt, mit Natriumhydrogencarbonatlösung neutral gewaschen und anschließend im Vakuum destilliert.Es werden 37,5 g (Ausb.: 75 % d.Th.) Produkt mit folgender Zusammensetzung erhalten (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 37,2 | 24,4 |
| Z-Isomer | 12,9 | 9,4 |
| Summe | 50,1 | 33,8 |

### Beispiel 5:

### Reaktionsbedingungen: Katalysator: Montmorillonit K 10 (33 Gew.-%); kein Lösungsmittel; Temperatur: 120°C, Reaktionszeit: 8 Stunden

30 g (E,Z)-8-Cyclohexadecen-1-on und 10 g Montmorillonit K 10 werden 8 Stunden bei 120°C erhitzt und anschließend im Vakuum destilliert. Es werden 21 g (Ausb.:70 % d.Th.) Produkt mit folgender Zusammensetzung erhalten (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 49,4 | 22,5 |
| Z-Isomer | 17,1 | 9,2 |
| Summe | 66,5 | 31,7 |

### Beispiel 6:

### Reaktionsbedingungen: Katalysator: Lewatit K 2641 (20 Gew.-%), kein Lösungsmittel; Temperatur: 120°C, Reaktionszeit: 8 Stunden

40 g (E,Z)-8-Cyclohexadecen-1-on und 8 g Lewatit K 2641 werden 8 Stunden bei 120°C erhitzt und anschließend im Vakuum destilliert. Es werden 22 g (Ausb.: 55 % d.Th.) Produkt mit folgender Zusammensetzung erhalten (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 54,2 | 7,1 |
| Z-Isomer | 30,1 | 6,3 |
| Summe | 84,3 | 13,4 |

### Beispiele 7 - 11: Umisomerisierung mit Katalysatoren auf Basis von Metallen der VIII. Nebengruppe

### Beispiel 7

### Reaktionsbedingungen: Katalysator: Iridium(I)-bis-(triphenylphosphin)-carbonyl-chlorid (1 Gew.-%), kein Lösungsmittel; Temperatur: 120°C, Reaktionszeit: 8 Stunden

40 g (E,Z)-8-Cyclohexadecen-1-on werden mit 0,4 g Iridium(I)-bis-(triphenylphosphin)-carbonyl-chlorid 8 Stunden bei 120°C erhitzt und anschließend in einer Claisendestillationsapparatur destilliert. Man erhält 35,9 g (Ausb.: 90 % d.Th.) Produkt folgender Zusammensetzung (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 43,4 | 28,5 |
| Z-Isomer | 19,7 | 5,5 |
| Summe | 63,1 | 34,0 |

### Beispiel 8

### Reaktionsbedingungen: Katalysator: Palladium(II)-bisbenzonitril-chlorid (1 Gew.-%), kein Lösungsmittel; Temperatur: 120°C, Reaktionszeit: 8 Stunden

40 g (E,Z)-8-Cyclohexadecen-1-on werden mit 0,4 g Palladium(II)-bisbenzonitril-chlorid 8 Stunden bei 120°C erhitzt und anschließend in einer Claisendestillationsapparatur destilliert. Man erhält 36,3 g (Ausb.: 91 % d.Th.) Produkt folgender Zusammensetzung (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 43,0 | 19,1 |
| Z-Isomer | 15,8 | 6,4 |
| Summe | 58,8 | 25,5 |

### Beispiel 9

### Reaktionsbedingungen: Katalysator: Rhodium(III)-chlorid Hydrat (3 Gew.-%), kein Lösungsmittel; Temperatur: 120°C, Reaktionszeit: 8 Stunden

40 g (E,Z)-8-Cyclohexadecen-1-on werden mit 1,2 g Rhodium(III)-chlorid Hydrat 8 Stunden bei 120°C erhitzt und anschließend in einer Claisendestillationsapparatur destilliert. Man erhält 38 g (Ausb.: 95 % d.Th.) Produkt folgender Zusammensetzung (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 55,1 | 16,3 |
| Z-Isomer | 20,9 | 6,0 |
| Summe | 76,0 | 22,3 |

### Beispiel 10

### Reaktionsbedingungen: Katalysator: Ruthenium(II)-tris-(triphenylphosphin)-chlorid (1 Gew.-%), kein Lösungsmittel; Temperatur: 120°C, Reaktionszeit: 5 Stunden

30 g (E,Z)-8-Cyclohexadecen-1-on werden mit 0,3 g Ruthenium(II)-tris-(triphenylphosphin)-chlorid 5 Stunden bei 120°C erhitzt und anschließend in einer Kugelrohrdestillationsapparatur destilliert. Man erhält 28,5 g (Ausb.: 95 % d.Th.) Produkt folgender Zusammensetzung (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 41,6 | 31,5 |
| Z-Isomer | 17,7 | 6,2 |
| Summe | 59,3 | 37,7 |

### Beispiel 11

### Reaktionsbedingungen: Katalysator: Ruthenium(II)-tris - (triphenylphosphin)-chlorid (0,1 Gew.-%), kein Lösungsmittel; Temperatur: 150°C, Reaktionszeit: 1 Stunde

30 g (E,Z)-8-Cyclohexadecen-1-on werden mit 0,03 g Ruthenium(II)-tris - (triphenylphosphin)-chlorid 1 Stunde bei 150°C erhitzt und anschließend in einer Kugelrohrdestillationsapparatur destilliert. Man erhält 29,4 g (Ausb.: 98 % d.Th.) Produkt folgender Zusammensetzung (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 42,7 | 30,0 |
| Z-Isomer | 16,4 | 8,1 |
| Summe | 59,1 | 38,1 |

### Beispiel 12

### Reaktionsbedingungen: Katalysator: Palladium auf Aktivkohle (2 Gew. -%, Pd-Gehalt: 5%, Wassergehalt 60%, entsprechend einer Palladium-Konzentration von 0,04 Gew.-%), kein Lösungsmittel; Temperatur: 170°C, Reaktionszeit: 23 Stunden

300 g (E,Z)-8-Cyclohexadecen-1-on werden mit 6 g Palladium (5% auf Aktivkohle, Wassergehalt 60%) 23 Stunden bei 170°C erhitzt und anschließend in einer Claisendestillationsapparatur destilliert. Man erhält 282 g (Ausb.: 94 % d.Th.) Produkt folgender Zusammensetzung (Angaben in Gew.-%, bezogen auf die Gesamtmasse des Produkts):

| | Cyclohexadec-**8-en**-1-on | Cyclohexadec-**7-en**-1-on |
|---|---|---|
| E-Isomer | 44,9 | 25,0 |
| Z-Isomer | 16,4 | 7,3 |
| Summe | 61,3 | 32,3 |

## Patentansprüche

1. Verfahren zur Herstellung eines (E,Z)-7,8-Cyclohexadecen-1-on-Isomerengemisches, mit folgendem Schritt:
- Kontaktieren von (E,Z)-8-Cyclohexadecen-1-on mit
(i) einer Säure ausgewählt aus der Gruppe bestehend aus Schwefelsäure und Sulfonsäuren und sauren Kationenaustauschern, oder
(ii) einem Katalysator enthaltend ein Element der VIII. Nebengruppe,
zum teilweisen Isomerisieren, so dass das (E,Z)-7,8-Cyclohexadecen-1-on-Isomerengemisch entsteht.

2. Verfahren nach Anspruch 1, wobei die Sulfonsäure ausgewählt ist aus der Gruppe bestehend aus Trifluormethansulfonsäure und p-Toluolsulfonsäure.

3. Verfahren nach Anspruch 1, wobei der saure Kationenaustauscher gewählt ist aus der Gruppe bestehend aus Lewatiten, Amberliten und Montmorilloniten.

4. Verfahren nach Anspruch 1, wobei das Element der VIII. Nebengruppe ausgewählt ist aus der Gruppe bestehend aus Ruthenium, Rhodium, Palladium und Iridium.

5. Verfahren nach Anspruch 1 oder 4, wobei das Element der VIII, Nebengruppe vorliegt
(a) in elementarer Form,
(b) in komplexierter oder unkomplexierter Form als Salz, wobei es eine formal ein- bis dreiwertige Oxidationsstufe besitzt,
oder
(c) als Komplexverbindung, wobei es formal nullwertig ist.

6. Verfahren nach einem der Ansprüche 4 oder 5, wobei das Element der VIII. Nebengruppe ausgewählt ist aus der Gruppe bestehend aus Rhodium(III)-chlorid Hydrat, Palladium(II)-bis-(benzonitril)-chlorid, Ruthenium(II)-tris-(triphenylphosphin)-chlorid, Iridium(I)-bis-(triphenylphosphin)-carbonyl-chlorid und Palladium auf Aktivkohle.

7. Verfahren nach einem der Ansprüche 1-5, wobei die Isomerisierung bei einer Temperatur im Bereich von 40 bis 250°C, vorzugsweise 80 bis 180°C, durchgeführt wird.

## Claims

1. Process for the preparation of an (E,Z)-7,8-cyclohexadecen-1-one isomer mixture, comprising the following step:
- bringing (E,Z)-8-cyclohexadecen-1-one into contact with
(i) an acid selected from the group comprising sulfuric acid, sulfonic acids and acidic cation exchangers, or
(ii) a catalyst containing an element of subgroup VIII,
for partial isomerization to form the (E,Z)-7,8-cyclohexadecen-1-one isomer mixture.

2. Process according to Claim 1 wherein the sulfonic acid is selected from the group comprising trifluoromethanesulfonic acid and p-toluenesulfonic acid.

3. Process according to Claim 1 wherein the acidic cation exchanger is selected from the group comprising Lewatits, Amberlites and montmorillonites.

4. Process according to Claim 1 wherein the element of subgroup VIII is selected from the group comprising ruthenium, rhodium, palladium and iridium.

5. Process according to Claim 1 or 4 wherein the element of subgroup VIII is present
(a) in elemental form,
(b) in complexed or uncomplexed form as a salt, its formal oxidation state being one to three,
or
(c) as a complex compound, its formal oxidation state being zero.

6. Process according to Claim 4 or 5 wherein the element of subgroup VIII is selected from the group comprising rhodium(III) chloride hydrate, bis(benzonitrile)-palladium(II) chloride, tris(triphenylphosphine)-ruthenium(II) chloride, bis(triphenylphosphine)carbonyl-iridium(I) chloride and palladium on activated charcoal.

7. Process according to one of Claims 1-5 wherein the isomerization is carried out at a temperature ranging from 40 to 250°C, preferably from 80 to 180°C.

## Revendications

1. Procédé de fabrication d'un mélange d'isomères (E,Z)-7,8-cyclohexadécèn-1-one, comprenant l'étape suivante:
- mise en contact de (E,Z)-8-cyclohexadécèn-1-one avec
(i) un acide choisi dans le groupe consistant en acide sulfurique et acide sulfonique et échangeurs de cations, ou
(ii) un catalyseur contenant un élément du groupe VIIIB,
pour l'isomérisation partielle, pour obtenir un mélange d'isomères cyclohexadécèn-1-one.

2. Procédé selon la revendication 1, dans lequel l'acide sulfonique est choisi dans le groupe constitué de l'acide trifluorométhanesulfonique et de l'acide p-toluènesulfonique.

3. Procédé selon la revendication 1, dans lequel l'échangeur de cations acide est choisi dans le groupe constitué de lewatites, amberlites et montmorillonites.

4. Procédé selon la revendication 1, dans lequel l'élément du groupe VIIIB est choisi dans le groupe constitué du ruthénium, du rhodium, du palladium et de l'iridium.

5. Procédé selon la revendication 1 ou 4, dans lequel l'élément du groupe VIIIB est présent
(a) sous une forme élémentaire
(b) sous une forme complexée ou non complexée en tant que sel, et possède un degré d'oxydation formel de monovalent à trivalent,
ou
(c) en tant que composé complexe de valence formelle nulle.

6. Procédé selon l'une des revendications 4 ou 5, l'élément du groupe VIIIB étant choisi dans le groupe constitué d'hydrate de chlorure de rhodium(III), de chlorure de palladium(II)-bis-(benzonitrile), de chlorure de ruthénium(II)-tris-(triphénylphosphine), de chlorure d'iridium(I)-bis-(triphénylphosphine)-carbonyle et de palladium sur charbon actif.

7. Procédé selon l'une des revendications 1 à 5, dans lequel l'isomérisation est effectuée à une température de 40 à 250°C, de préférence 80 à 180°C.
